(19) Europäisches Patentamt / European Patent Office / Office européen des brevets

(11) EP 1 524 259 B1

(12) EUROPÄISCHE PATENTSCHRIFT

(45) Veröffentlichungstag und Bekanntmachung des Hinweises auf die Patenterteilung:
**08.06.2016 Patentblatt 2016/23**

(51) Int Cl.:
***C07C 209/36*** (2006.01) ***C07C 211/46*** (2006.01)

(21) Anmeldenummer: **04023292.8**

(22) Anmeldetag: **30.09.2004**

(54) **Verfahren zur Herstellung von aromatischen Aminen durch heterogen katalysierte Hydrierung**

Process for the preparation of aromatic amines by heterogene catalyzed hydrogenation

Procédé pour la production de amines aromatiques par hydrogénation catalysé heterogene

(84) Benannte Vertragsstaaten:
**AT BE BG CH CY CZ DE DK EE ES FI FR GB GR HU IE IT LI LU MC NL PL PT RO SE SI SK TR**

(30) Priorität: **13.10.2003 DE 10347439**

(43) Veröffentlichungstag der Anmeldung:
**20.04.2005 Patentblatt 2005/16**

(73) Patentinhaber: **Covestro Deutschland AG**
**51373 Leverkusen (DE)**

(72) Erfinder:
- **Lehner, Peter Dr**
**40878 Ratingen (DE)**
- **Turek, Thomas Dr**
**40239 Düsseldorf (DE)**
- **Brandt, Matthias Dr.**
**42399 Wuppertal (DE)**
- **Buchholz, Susanne Dr**
**50825 Köln (DE)**

(74) Vertreter: **Levpat**
**c/o Covestro AG**
**Alfred-Nobel-Straße 10**
**40789 Monheim am Rhein (DE)**

(56) Entgegenhaltungen:
**EP-A- 0 978 505** **EP-A- 1 254 715**
**EP-A- 1 336 428**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die Erfindung betrifft ein Verfahren zur Herstellung von aromatischen Aminen durch katalytische Hydrierung von aromatischen Nitroverbindungen.

Aromatische Amine sind wichtige Zwischenprodukte, die preiswert und in großen Mengen hergestellt werden müssen. Daher werden Produktionsanlagen für aromatische Amine in der Regel für sehr große Kapazitäten errichtet. Die Hydrierung von Nitroaromaten ist eine stark exotherme Reaktion. Die Abführung und energetische Nutzung der Reaktionswärme ist daher ein wichtiger Aspekt bei der Herstellung von Nitroaromaten.

Für die Gasphasenhydrierung von Nitroaromaten eignen sich verschiedene Reaktoren. So wird z.B. in US-A 3 136 818 ein Verfahren beschrieben, bei dem die Reaktion in einem Wirbelbett durchgeführt wird. Der effektiven Wärmeabfuhr dieser Verfahrensweise stehen Probleme durch die uneinheitliche Verweilzeitverteilung (Durchbruch der Nitroaromaten) und durch Katalysatorabrieb gegenüber.

EP 0 978 505 A2 offenbart die mehrphasige adiabatische Hydrierung von Dinitrotoluol zu Toluoldiamin an einem monolithischen Katalysator.

Andere Verfahren nutzen ortsfeste Katalysatoren in Festbetten. Mit dieser Anordnung kann die Reaktion mit sehr enger Verweilzeitverteilung und unter Umgehung der Problematik des Katalysatorabriebs durchgeführt werden. Durch eine adiabate Betriebsweise des Festbettreaktors können Probleme bei der Wärmeabfuhr umgangen werden. Ein solches Verfahren, welches sich durch einen einfachen Aufbau und leichte Skalierbarkeit der einzelnen Apparate auszeichnet, wird z.B. in EP-A 0 696 574 beschrieben. Um den adiabaten Temperaturanstieg in Grenzen zu halten, müssen bei der adiabaten Betriebsweise allerdings sehr große Gasströme im Kreislauf geführt werden. In DE-A 28 49 002 wird ein Verfahren zur Reduktion von Nitroverbindungen in Gegenwart von ortsfesten, palladiumhaltigen Mehrkomponenten-Trägerkatalysatoren in gekühlten Rohrbündelreaktoren beschrieben. Der Kontakt besteht im wesentlichen aus 1 bis 20 g Palladium, 1 bis 20 g Vanadium und 1 bis 20 g Blei pro Liter $\alpha$-$Al_2O_3$. Dabei hat es sich als vorteilhaft erwiesen, wenn die aktiven Komponenten möglichst nah an der Oberfläche des Katalysators in einer sehr scharfen Zone niedergeschlagen vorliegen und im Inneren des Trägermaterials keine aktiven Komponenten enthalten sind. Nachteilig bei der in DE-A 28 49 002 beschriebenen Gasphasenhydrierung ist die geringe spezifische Belastung der Katalysatoren. Die angegebenen Belastungen betragen ca. 0.4 bis 0.5 kg/(l·h). Die Belastung ist dabei definiert als die Menge an Nitroaromaten in kg, die pro Liter Katalysatorschüttung innerhalb einer Stunde durchgesetzt wird. Verbunden mit der geringen Katalysatorbelastung ist eine nicht befriedigende Raum-Zeit-Ausbeute bei großtechnischen Verfahren zur Herstellung von aromatischen Aminen. Ferner sind die Selektivitäten zu Beginn einer Fahrperiode deutlich niedriger als gegen Ende, was zu Ausbeuteverlusten und Problemen bei der Aufarbeitung des Rohprodukts führt.

Die Belastung des Katalysators kann in isotherm betriebenen Reaktoren nur dann erhöht werden, wenn die bei der Reaktion freigesetzte Wärme effizient abgeführt werden kann. In WO 98/25881 wird der Einsatz von inerten Materialien zur Verdünnung der Katalysatorschüttung bei der Herstellung von aromatischen Aminen beschrieben. Durch die Verdünnung wird die Reaktionszone verbreitert und somit die für den Wärmeaustausch zur Verfügung stehende Fläche vergrößert. Mit dieser Vorgehensweise kann die hot spot-Temperatur abgesenkt werden bzw. die mögliche Nitroaromaten-Belastung bei konstanter hot spot-Temperatur erhöht werden. Durch die Verdünnung sinkt allerdings die Standzeit der Schüttung ab. Bei dem in WO 98/25881 aufgeführten Beispiel lag die Produktivität der verdünnten Schüttung aufgrund der kurzen Standzeiten trotz höherer Belastung deutlich unter der Produktivität der unverdünnten Schüttung.

In einer anderen Verfahrensvariante wird die Hydrierung von Nitroaromaten in thermostatisierten Rohrbündelreaktoren durchgeführt. Als Katalysatoren werden u.a. geträgerte Kupfer- oder Palladium-Katalysatoren eingesetzt. In GB-A 1 452 466 wird ein Verfahren zur Herstellung von Anilin in einem thermostatisierten Rohrbündelreaktor unter Nutzung eines geträgerten Kupfer-Katalysators beschrieben. Zur Umsatzvervollständigung wird dort eine Katalysatorschüttung als adiabater Nachreaktor eingesetzt.

In DE-A 199 31 902 wird ein Verfahren zur Herstellung von monolithischen Oxidationskatalysatoren und deren Verwendung bei der Gasphasenoxidation von Kohlenwasserstoffen beschrieben. Hier wird ein monolithischer Wabenkatalysator als adiabater Reaktor dem isotherm betriebenen Hauptreaktor nachgeschaltet.

Die Aufgabe der vorliegenden Erfindung besteht darin, ein Verfahren zur Herstellung von aromatischen Aminen durch katalytische Hydrierung von aromatischen Nitroverbindungen zur Verfügung zu stellen, welches im technischen Maßstab durchführbar ist und gegenüber den aus dem Stand der Technik bekannten, in Rohrbündelreaktoren durchgeführten Verfahren eine höhere Raum-Zeit-Ausbeute und längere Standzeit ermöglicht.

Gegenstand der Erfindung ist ein Verfahren zur Herstellung von aromatischen Aminen durch katalytische Hydrierung von aromatischen Nitroverbindungen, welches dadurch gekennzeichnet ist, dass die katalytische Hydrierung mindestens einstufig ausgeführt wird und der Katalysator wenigstens aus einem monolithischen Träger und einer katalytisch aktiven Beschichtung besteht.

Der Begriff Monolith, wie er im Sinne der vorliegenden Erfindung verwendet wird, wird z.B. in "Monoliths in multiphase catalytic processes - aspects and prospects" von F. Kapteijn, J. J. Heiszwolf, T. A. Nijhuis und J. A. Moulijn, Cattech 3, 1999, S. 24 definiert. Danach versteht man unter Monolithen nicht nur die "klassischen" Trägerkörper mit

parallelen, radial nicht untereinander verbundenen Kanälen. Es zählen auch Schäume, Schwämme o.dgl. mit dreidimensionalen Verbindungen innerhalb des Trägerkörpers zu den Monolithen sowie Trägerkörper mit Kreuzstromkanälen.

Der druckverlustarme, monolithische Träger kann eine Wabenstruktur, aber auch eine offene oder geschlossene Kreuzkanalstruktur aufweisen. Der monolithische Träger besitzt eine bevorzugte Zelldichte von 100 bis 900 cpsi (cells per square inch), besonders bevorzugt von 200 bis 600 cpsi.

Als Materialien für den monolithischen Träger eignen sich keramische Werkstoffe wie z.B. Cordierit, Silicate, Siliziumdioxid, Silziumcarbid, Aluminiumoxid, Aluminate, Mullite oder Mischungen aus diesen Stoffen sowie Metalle und Metalllegierungen.

Der monolithische Träger kann mit gängigen, aus dem Stand der Technik bekannten Verfahren mit einer katalytisch aktiven Beschichtung versehen werden. Bevorzugt wird zur Beschichtung ein Verfahren eingesetzt, das als Dip-Coating bezeichnet wird. Das Verfahren wird beispielsweise in "Preparation of monolithic catalysts" von T. A. Nijhuis, A. E. W. Beers, T. Vergunst, I. Hoek, F. Kapteijn und J. A. Moulijn in Catalysis Reviews, Band 43, 2001, Seite 345 - 380 beschrieben. Der monolithische Träger wird dabei mit einer Suspension beschichtet, die auf einer feinst aufgemahlenen, $Al_2O_3$-geträgerten katalytisch aktiven Komponente basiert.

Bevorzugt enthält die katalytisch aktive Beschichtung für die Hydrierung von aromatischen Nitroverbindungen in der Gasphase Metalle aus den Gruppen VIIIa, Ib, IIb, IVa, Va, VIa, IVb und Vb des Periodensystems der Elemente (Mendelejew) als katalytisch aktive Komponente. Bevorzugte Metalle sind z.B. Pd, Pt, Cu oder Ni. Die katalytisch aktive Komponente kann geträgert sein. Als Trägersubstanz eignen sich keramische Stoffe, wie z.B. $Al_2O_3$, $SiO_2$, $TiO_2$ oder Zeolithe, aber auch Graphit oder Kohlenstoff. Die Trägersubstanz ist vorzugsweise fein gemahlen. Besonders bevorzugt wird der in DE-A 28 49 002 beschriebene Katalysator als katalytisch aktive Beschichtung eingesetzt.

Um eine gleichmäßige Beschichtung zu erreichen, sollte die volumenbezogene Partikelgröße $d_{90,3}$ der vorzugsweise aufgemahlenen Trägersubstanz bevorzugt kleiner als 50 $\mu$m, besonders bevorzugt kleiner als 10 $\mu$m, sein.

Der besondere Vorteil des Dip-Coating-Verfahrens ist, dass eine dünne Schicht einer katalytisch aktiven Komponente vergleichsweise einfach auf einen monolithischen Träger aufgebracht werden kann. Mit dem Dip-Coating-Verfahren kann eine katalytisch aktive Beschichtung mit einer Schichtdicke von maximal 250 $\mu$m, vorzugsweise von maximal 100 $\mu$m, besonders bevorzugt von 10 bis 100 $\mu$m, auf den monolithischen Träger aufgebracht werden. Das Dip-Coating kann ein- oder mehrmals durchgeführt werden. Durch mehrmaliges Beschichten können monolithische Träger mit einer katalytisch aktiven Masse, bestehend aus Trägersubstanz und katalytisch aktiver Komponente, insbesondere bis maximal 150 g/l, bevorzugt von 30 bis 150 g/l, besonders bevorzugt von 50 bis 120 g/l, monolitischem Träger hergestellt werden.

Die vorliegende Anmeldung betrifft ein Verfahren zur Herstellung von aromatischen Aminen durch katalytische Hydrierung von aromatischen Nitroverbindungen ausgewählt aus Nitrobenzol und den isomeren Nitrotoluolen, dadurch gekennzeichnet, dass die katalytische Hydrierung mindestens einstufig ausgeführt wird und der Katalysator wenigstens aus einem monolithischen Träger und einer katalytisch aktiven Beschichtung besteht, wobei die Temperatur des Eduktgasgemisches vor dem Reaktoreingang bei 200 bis 400 °C liegt und wobei die Hydrierung in einem adiabatisch betriebenen Reaktor, dem Wasserstoff und aromatische Nitroverbindung in einem molaren Verhältnis von Wasserstoff zu Nitrogruppe von 3:1 bis 100:1 zugeführt werden, bei einer Temperatur von 200 bis 500 °C stattfindet.

Das erfindungsgemäße Verfahren kann in technischem Maßstab durchgeführt werden und weist gegenüber den aus dem Stand der Technik bekannten, insbesondere in Rohrbündelreaktoren durchgeführten, Verfahren eine höhere Raum-Zeit-Ausbeute und längere Standzeit auf.

Der erfindungsgemäß zur Herstellung von aromatischen Aminen eingesetzte Katalysator, bestehend aus einem monolithischen Träger und einer katalytisch aktiven Beschichtung, zeigt gegenüber herkömmlichen, aus dem Stand der Technik bekannten Katalysatorschüttungen wesentliche Vorteile. Zum einen ist der Druckverlust von monolithischen Trägern bei vergleichbarer Durchströmungsgeschwindigkeit wesentlich geringer als der von Katalysatorschüttungen. Umgekehrt kann der monolithische Träger bei gleichem Druckverlust mit weitaus höheren Geschwindigkeiten durchströmt werden. Infolge des geringen Druckverlusts auch bei sehr hohen Strömungsgeschwindigkeiten ist die Verwendung monolithischer Träger zum Beispiel für den Einsatz in nachgeschalteten Reaktoren oder bei Verfahren, die sich durch hohe Volumenströme und Strömungsgeschwindigkeiten auszeichnen, vorteilhaft. Dabei können mit diesen Katalysatoren wesentlich kompaktere Reaktoren gebaut werden.

Ein weiterer Vorteil beruht auf der sehr dünnen katalytisch aktiven Beschichtung. Sind die katalytisch aktiven Komponenten in einer sehr dünnen Schicht abgeschieden, ist der Diffusionseinfluss weitaus geringer als bei Vollkatalysatoren. Wird die Hauptreaktion von Folgereaktionen begleitet, kann mit diesen sehr dünnen katalytisch aktiven Beschichtungen eine höhere Selektivität erreicht werden. Das Aufbringen in einer dünnen Schicht kann außerdem Vorteile bezüglich der Wertproduktselektivität mit sich bringen.

Das erfindungsgemässe Verfahren wird vorzugsweise bei Drücken von 1 bis 30 bar, besonders bevorzugt von 1 bis 20 bar, ganz besonders bevorzugt von 1 bis 15 bar, betrieben. Die Temperatur des Eduktgasgemischs liegt vor dem Reaktoreingang vorzugsweise bei 200 bis 400 DEG C, die Temperatur im Katalysatorbett liegt bevorzugt bei 200 bis 500 DEG C. Wasserstoff und aromatische Nitroverbindung werden dem Reaktor in einem molaren Verhältnis von

Wasserstoff zu Nitrogruppe von vorzugsweise 3:1 bis 100:1 zugeführt. Als aromatische Nitroverbindung können insbesondere solche der nachstehenden Formel hydriert werden:

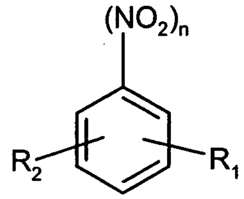

wobei $R_1$ und $R_2$ gleich oder verschieden sind und für Wasserstoff oder $C_1$- bis $C_4$-Alkyl, insbesondere Methyl und Ethyl, stehen und n 1 oder 2 bedeutet. Bevorzugt werden Nitrobenzol oder die isomeren Nitrotoluole nach dem erfindungsgemäßen Verfahren hydriert.

Zur Durchführung des erfindungsgemäßen Verfahrens kann der Katalysator z.B. in einem adiabaten Reaktor, wie er für die Herstellung von aromatischen Aminen gemäß EP-B 0 696 574 beschrieben ist, eingesetzt werden. Der Einsatz monolithischer Träger nach dem erfindungsgemäßen Verfahren ist grundsätzlich auch in thermostatisierten Reaktoren möglich. Allerdings ist der Einsatz solcher monolithischer Träger in thermostatisierten Reaktoren aufgrund der schlechten radialen Wärmeableitung, insbesondere im technischen Maßstab, in der Regel wenig vorteilhaft.

Gemäß EP-B 0 696 574 wird für die adiabatische Reaktionsführung bei der Hydrierung von Nitroaromaten ein Wasserstoff/Nitrogruppen-Verhältnis von 60:1 bis 800:1 angestrebt. Da je Nitrogruppe nur 3 Äquivalente Wasserstoff bei der Hydrierung verbraucht werden, wird die Reaktion mit einem sehr großen Wasserstoff-Überschuss gefahren. Aufgrund des hohen Wasserstoff-Überschusses werden die Gasvolumenströme in kommerziellen Produktionsanlagen sehr groß. Daher ist ein geringer Druckverlust im Reaktor ein wichtiges Kriterium bei der Auswahl des Katalysators. Monolithische Träger, z.B. Wabenkörper, weisen hierbei einen wesentlichen Vorteil gegenüber einer vergleichbaren Katalysatorschüttung auf.

Die Herstellung von aromatischen Aminen kann nach dem erfindungsgemäßen Verfahren auch in einem zwei- oder mehrstufigen Verfahren durchgeführt werden. In der zweistufigen Ausführungsform des Verfahrens, wird die Reaktion z.B. zunächst in einem isotherm betriebenen, thermostatisierten Hauptreaktor mit einer ortsfesten Katalysatorschüttung durchgeführt, wobei der Umsatzgrad 80 % bis 100 % betragen kann. Wie z.B. in DE-A 28 49 002 und WO 98/46557 erwähnt, wird die Hydrierung von Nitroaromaten von einer Verkokung der Katalysatoren begleitet. Bei Reaktoren mit ortsfester Katalysatorschüttung führt dies zu einem Durchbrechen der Schüttung. Ab diesem Zeitpunkt können die Nitroaromaten aufgrund der Desaktivierung der Katalysatorschüttung nicht mehr zu 100 % umgesetzt werden. Der nachgeschaltete, adiabat betriebene Nachreaktor wird dann zur Vervollständigung des Umsatzes auf 100 % genutzt, wenn der Umsatzgrad des Hauptreaktors kleiner als 100 % ist. Durch den Einsatz des adiabaten Nachreaktors lässt sich die Standzeit des isothermen Hauptreaktors erhöhen. Besonders vorteilhaft ist die vorliegende Erfindung in Zusammenhang mit der Nachrüstung von bestehenden thermostatisierten Rohrbündelreaktoren mit dem Ziel der Steigerung der Raum-Zeit-Ausbeute.

In einer weiteren bevorzugten Ausführungsform ist das Verfahren zweistufig, wobei in der ersten Stufe ein isotherm betriebener, thermostatisierter Hauptreaktor mit einer ortsfesten Katalysatorschüttung und in der zweiten Stufe ein adiabat betriebener Nachreaktor mit einem Katalysator, umfassend einen monolitischen Träger, eingesetzt wird. Dabei ist die Katalysatorschüttung in dem Hauptreaktor mit Inertmaterial verdünnt. Die Verdünnung der Katalysatorschüttung mit Inertmaterial erlaubt eine Erhöhung der Katalysatorbelastung, wie z.B. in WO 98/46557 beschrieben. Als Verdünnungsmaterial (Inertmaterial) können z.B. der inerte Katalysatorträger der Katalysatorschüttung oder andere inerte Füllkörper aus Glas, Keramik oder Metall eingesetzt werden. Bevorzugt werden Stoffe mit hoher Wärmeleitfähigkeit eingesetzt. Üblicherweise wird der Katalysator mit 10 bis 50 Vol.-% Inertmaterial, bevorzugt mit 20 bis 40 Vol.-%, Inertmaterial verdünnt. Allerdings sinkt durch die Verdünnung der Schüttung die Standzeit des Reaktors, da sich weniger aktiver Katalysator im Reaktionsvolumen befindet. Dieser Standzeitverlust kann durch den Einsatz eines adiabat betriebenen Nachreaktors wieder kompensiert werden. Durch die Kombination von verdünnter Katalysatorschüttung in einem Hauptreaktor und adiabatem Nachreaktor kann die Produktivität des Reaktors gesteigert werden. Ganz besonders eignet sich dieses Verfahren für bestehende Reaktoren. Durch die Verwendung des monolithischen Trägers mit sehr geringem Druckverlust kann der Nachreaktor problemlos in den bestehenden Reaktionskreislauf eingebunden werden. Mit dem monolithischen Träger, z.B. Wabenkörper, können im Nachreaktor z.B. auch Strömungsgeschwindigkeiten von mehr als 10 m/s realisiert werden. Dadurch ergibt sich die Möglichkeit, die Verweilzeiten im Nachreaktor auf Werte weniger als 1 s abzusenken. Daher wird die Selektivität des Hauptreaktors durch Folgereaktionen im Nachreaktor kaum beeinflusst. Somit kann die Kapazität bestehender Reaktoren für die Hydrierung von Nitroaromaten durch die Nachrüstung eines adiabaten Nachreaktors mit monolithischem Katalysator mit sehr geringem Aufwand gesteigert werden.

**Beispiele:**

**Beispiel 1: Herstellung katalytisch beschichteter monolithischer Wabenkörper**

Als monolithische Träger wurden zylindrische Wabenkörper aus Cordierit mit einer Länge von 152 mm, einem Durchmesser von 30.5 mm und einer Zelldichte von 400 cpsi verwendet. Die monolithischen Träger wurden vor der Beschichtung gereinigt, getrocknet und gewogen. Die Zusammensetzung der geträgerten katalytisch aktiven Komponenten als katalytisch aktive Beschichtung war wie folgt: 9 g Palladium, 9 g Vanadium und 3 g Blei je Liter kugelförmigen alpha-Aluminiumoxids (eine detaillierte Beschreibung des Trägerkatalysators findet sich in DE-A 28 49 002). Aus diesen geträgerten katalytisch aktiven Komponenten wurde vor der Beschichtung durch Zerkleinerung und Feinstmahlung ein Pulver mit einer Partikelgröße von $d_{50,3}$ = 1,7 $\mu$m und $d_{90,3}$ = 4,9 $\mu$m erzeugt.

Für die Beschichtungs-Suspension wurde eine Lösung aus 3,2 g 25 gew.-%iger wässriger Ammoniumpolymethacrylat-Lösung ("Darvan C", Firma R. T. Vanderbilt) und 116,8 g vollentsalztem Wasser angesetzt, in die anschließend 80,0 g des feinstgemahlenen Katalysators eingemischt wurden. Der Feststoffgehalt der Suspension betrug somit ca. 40 Gew.-%. In diese Suspension wurde dann für einige Minuten ein Wabenkörper eingetaucht. Nach dem Herausnehmen aus der Suspension ließ man den Wabenkörper abtropfen und blies dann mit einer Druckluftpistole die Kanäle frei. Anschließend erfolgten eine Trocknung (2 h bei 120°C im Umluft-Trockenschrank) und eine Calcinierung (2 h bei 500°C im Muffelofen). Danach wurde der beschichtete Wabenkörper erneut gewogen.

Die Beladung des monolithischen Trägers mit der katalytisch aktiven Masse, bestehend aus Trägersubstanz und katalytisch aktiver Komponente, betrug 68 g/l, die Schichtdicke der katalytisch aktiven Beschichtung betrug maximal 100 $\mu$m.

Auf die beschriebene Art und Weise wurden insgesamt sieben Wabenkörper mit Katalysator beschichtet. Die dabei erzielten Katalysatorbeladungen $w_K$, errechnet nach der Formel

$$w_K = \frac{\text{Masse}_{\text{Wabenkörper nach Beschichtung}} - \text{Masse}_{\text{Wabenkörper vor Beschichtung}}}{\text{Masse}_{\text{Wabenkörper nach Beschichtung}}}$$

betrugen von 10 bis 14 Gew.-%.

**Beispiel 2: Standard-Schüttung (Vergleichsbeispiel)**

Ein mit Öl thermostatisierter Rohrreaktor mit einem Innendurchmesser von 26 mm und einer Länge von 3000 mm wurde mit einem Katalysator, wie er in DE-A 28 49 002 beschrieben ist, gefüllt. Im Zentrum des Rohrreaktors befand sich ein Schutzrohr mit einem beweglichen Thermoelement zur Erfassung der Temperatur in der Katalysatorschüttung. Der Katalysator wurde zuerst mit Stickstoff, dann mit Wasserstoff gespült und dann über einen Zeitraum von 49 h mit 1000 1/h Wasserstoff bei 240°C aktiviert.

Anschließend wurde Nitrobenzol in einem Gemisch aus Stickstoff und Wasserstoff verdampft. Die Nitrobenzolbelastung wurde langsam von 134 g/h bis zum Maximalwert von 690 g/h so erhöht, dass die Maximaltemperatur in der Schüttung nicht über 460°C anstieg. In dieser Phase wurde das Reaktionsrohr mit einem Volumenstrom des Gemisches von 820 l/h durchströmt, wobei ein Molverhältnis Wasserstoff zu Nitrobenzol von 4 zu 1 eingehalten wurde. Nach Erreichen der maximalen Nitrobenzolmenge wurde der Stickstoff komplett durch Wasserstoff ersetzt und das Reaktionsrohr wurde dann mit 820 l/h Wasserstoff beaufschlagt. Die Öltemperatur wurde während der gesamten Reaktionsdauer konstant bei 240°C gehalten.

Im Bereich der maximalen Nitrobenzolbelastung wanderte der hot spot mit einer Geschwindigkeit von ca. 1 mm/h durch die Schüttung. Bei maximaler Nitrobenzolbelastung lag die maximale Temperatur bei 435°C. Die Selektivität betrug 98.7 % nach 47 h, 99.5 % nach 119 h und 99.5 % nach 408 h.

**Beispiel 3: Einsatz des monolithischen Wabenkörpers (Ausführungsbeispiel)**

In einen mit Öl thermostatisierten Rohrreaktor mit einem Innendurchmesser von 32.8 mm wurden gemäß Beispiel 1 hergestellte monolithische Wabenkörper als Katalysatoren eingesetzt. In das Reaktionsrohr wurden 5 Wabenkörper mit einer Länge von jeweils 150 mm und einem Durchmesser von 30.5 mm eingebracht. Die auf diesen 5 Wabenkörpern abgeschiedene aktive Katalysatormasse, also Trägersubstanz und katalytisch aktive Komponente, betrug insgesamt 37.5 g. Der Spalt zwischen der Rohrwand und dem Wabenkörper wurde mit einem hitzebeständigen Fleece (Fa. Carborundum, Typ FT 1) abgedichtet. Im Zentrum des Rohrreaktors befand sich ein Schutzrohr mit einem beweglichen Thermoelement zur Erfassung der Temperatur in dem monolithischen Wabenkörper. Der Katalysator wurde

zuerst mit Stickstoff, dann mit Wasserstoff gespült und schließlich über einen Zeitraum von 3 h mit 500 l/h Wasserstoff bei 300°C aktiviert.

Anschließend wurde Nitrobenzol in einem Gemisch aus Stickstoff und Wasserstoff verdampft. Die Nitrobenzolbelastung wurde innerhalb von 72 h schrittweise von 30 g/h bis auf 110 g/h erhöht. Nach Erreichen einer Nitrobenzolmenge 110 g/h wurde der Stickstoff komplett durch Wasserstoff ersetzt. Das Reaktionsrohr wurde dann mit 140 l/h Wasserstoff beaufschlagt. Die Öltemperatur wurde während der gesamten Reaktionsdauer konstant bei 240°C gehalten. Die Selektivität lag hier bereits nach 28 h bei über 99.9 %. Mit dem monolithischen Wabenkörper können somit im Vergleich zur Schüttung (siehe Beispiel 2) deutlich höhere Selektivitäten erreicht werden. Diese hohe Selektivität wird zudem schneller erreicht.

**Beispiel 4: Einsatz des monolithischen Katalysators zur Umsatzvervollständigung (Mischungen aus Anilin, Wasser und Nitrobenzol)**

In einen mit Öl thermostatisierten Rohrreaktor mit einem Innendurchmesser von 32.8 mm wurden gemäß Beispiel 1 hergestellte monolithische Wabenkörper als Katalysatoren eingesetzt. In das Reaktionsrohr wurden 5 Wabenkörper mit einer Länge von jeweils 150 mm und einem Durchmesser von 30.5 mm eingebracht. Die auf diesen 5 Wabenkörpern abgeschiedene aktive Katalysatormasse, also Trägersubstanz und katalytisch aktiver Komponente, betrug insgesamt 37.5 g. Der Spalt zwischen der Rohrwand und dem Wabenkörper wurde mit einem hitzebeständigen Fleece (Fa. Carborundum, Typ FT 1) abgedichtet. Im Zentrum des Rohrreaktors befand sich ein Schutzrohr mit einem beweglichen Thermoelement zur Erfassung der Temperatur in dem monolithischen Wabenkörper. Der Katalysator wurde zuerst mit Stickstoff, dann mit Wasserstoff gespült und schließlich über einen Zeitraum von 24 h mit 700 l/h Wasserstoff bei 300°C aktiviert.

Anschließend wurden Anilin und Wasser im Molverhältnis 1 zu 2 in einen Wasserstoffstrom verdampft. Dem Wasser wurde Nitrobenzol hinzugegeben, wobei der Anteil des Nitrobenzols bezogen auf das eingesetzte Anilin variiert wurde. Die durchgesetzte Menge an Anilin, Wasser, Nitrobenzol und Wasserstoff wurden in einem breiten Betriebsfenster variiert (siehe Tabelle 1). Die Öltemperatur wurde während der gesamten Reaktionsdauer konstant bei 300°C gehalten. In allen Fällen konnte das eingesetzte Nitrobenzol mit den eingesetzten monolithischen Wabenkörpern nahezu vollständig umgesetzt werden.

Tabelle 1: Parameter der durchgeführten Versuche in der Schüttung

| | Fall 1 | Fall 2 | Fall 3 | Fall 4 |
|---|---|---|---|---|
| Anilin | 612 g/h | 612 g/h | 612 g/h | 1448 g/h |
| Nitrobenzol | 0.1 g/h | 0.6 g/h | 66.6 g/h | 257 g/h |
| Wasserstoff | 450 l/h | 450 l/h | 1685 l/h | 1685 l/h |
| Nitrobenzol im Edukt | 19 ppm | 998 ppm | 9.8 Gew.-% | 15 Gew.-% |
| Nitrobenzol im Produkt | 5 ppm | 3 ppm | 1 ppm | 3 ppm |

**Beispiel 5: Verdünnte Schüttung (Ausführungsbeispiel)**

In den in Beispiel 2 beschriebenen Rohrreaktor wurde eine Mischung aus 50 Vol.-% des in DE-A 28 49 002 beschriebenen Katalysators und 50 Vol.-% SiC (SIKA I F8 der Firma Norton SIKA) zur Verdünnung der Katalysatorschüttung eingebracht. Die Schüttung wurde, wie in Beispiel 2 beschrieben, mit Wasserstoff aktiviert.

a) Anschließend wurde die Nitrobenzolbelastung, wie in Beispiel 2 beschrieben, schrittweise auf eine maximale Menge von 732 g/h erhöht. Ab Erreichen der maximalen Nitrobenzolmenge wurde die Schüttung mit 852 l/h Wasserstoff beaufschlagt. Die Selektivität betrug 98.0 % nach 52 h, 98.5 % nach 121 h und 99.5 % nach 409 h.

b) Analog zu a) wurde bei derselben Schüttung eine maximale Nitrobenzolmenge von 1236 g/h und 1460 l/h Wasserstoff eingesetzt. Die Selektivität betrug 92.2 % nach 48 h, 98.3 % nach 123 h und 99.7 % nach 408 h.

c) Analog zu a) wurde bei derselben Schüttung eine maximale Nitrobenzolmenge von 2028 g/h und 2211 l/h Wasserstoff eingesetzt. Die Selektivität betrug 98.4 % nach 50 h, 99.4 % nach 124 h und 99.7 % nach 220 h.

In allen Fällen wurden im Bereich des hot spots, im Vergleich zur unverdünnten Schüttung (Beispiel 2), geringere

Temperaturen gemessen. Aufgrund der höheren spezifischen Katalysatorbelastung wandert der hot spot aber in allen Beispielen schneller durch die Schüttung als dies in Beispiel 2 der Fall ist (siehe Tabelle 2).

Tabelle 2: Parameter der durchgeführten Versuche in der Schüttung

| | Beispiel 2 | Beispiel 5a | Beispiel 5b | Beispiel 5c |
|---|---|---|---|---|
| Nitrobenzol | 690 g/h | 732 g/h | 1236 g/h | 2028 g/h |
| Wasserstoff | 820 l/h | 852 l/h | 1460 l/h | 2211 l/h |
| Wanderungsgeschwindigkeit | 1 mm/h | 1,7 mm/h | 2,3 mm/h | 4,4 mm/h |
| $T_{hot\ spot,max}$ | 435°C | 332°C | 370°C | 401°C |

## Patentansprüche

**1.** Verfahren zur Herstellung von aromatischen Aminen durch katalytische Hydrierung von aromatischen Nitroverbindungen ausgewählt aus Nitrobenzol und den isomeren Nitrotoluolen, **dadurch gekennzeichnet, dass** die katalytische Hydrierung mindestens einstufig ausgeführt wird und der Katalysator wenigstens aus einem monolithischen Träger und einer katalytisch aktiven Beschichtung besteht, wobei die Temperatur des Eduktgasgemisches vor dem Reaktoreingang bei 200 bis 400 °C liegt und wobei die Hydrierung in einem adiabatisch betriebenen Reaktor, dem Wasserstoff und aromatische Nitroverbindung in einem molaren Verhältnis von Wasserstoff zu Nitrogruppe von 3:1 bis 100:1 zugeführt werden, bei einer Temperatur von 200 bis 500 °C stattfindet.

**2.** Verfahren nach Anspruch 1, **dadurch gekennzeichnet, dass** die Zelldichte des monolithischen Trägers 100 bis 900 cpsi, vorzugsweise 200 bis 600 cpsi, beträgt.

**3.** Verfahren nach einem der Ansprüche 1 oder 2, **dadurch gekennzeichnet, dass** die katalytisch aktive Beschichtung eine Schichtdicke von maximal 250 μm, vorzugsweise von maximal 100 μm, besonders bevorzugt von 10 bis 100 μm, aufweist.

**4.** Verfahren nach einem der Ansprüche 1-3, **dadurch gekennzeichnet, dass** die katalytisch aktive Beschichtung Platin und/oder Palladium enthält.

**5.** Verfahren nach einem der Ansprüche 1-4, **dadurch gekennzeichnet, dass** die katalytisch aktive Beschichtung geträgertes Platin und/oder Palladium enthält.

**6.** Verfahren nach einem der Ansprüche 1-5, **dadurch gekennzeichnet, dass** die katalytische Hydrierung bei einem Druck von 1 bis 30 bar, bevorzugt 1 bis 20 bar, besonders bevorzugt 1 bis 15 bar, stattfindet.

**7.** Verfahren nach einem der Ansprüche 1-6, **dadurch gekennzeichnet, dass** die katalytische Hydrierung zweistufig ausgeführt wird, wobei in der zweiten Stufe ein adiabatisch betriebener Reaktor mit einem Katalysator eingesetzt wird, welcher wenigstens aus einem monolithischen Träger und einer katalytisch aktiven Beschichtung besteht.

## Claims

**1.** Process for the production of aromatic amines by catalytic hydrogenation of aromatic nitro compounds selected from nitrobenzene and the isomeric nitrotoluenes, **characterised in that** the catalytic hydrogenation is carried out in at least one step and the catalyst consists at least of a monolithic support and a catalytically active coating, wherein the temperature of the feed gas mixture before entering the reactor is 200 to 400°C and wherein the hydrogenation takes place at a temperature of 200 to 500°C in an adiabatically operated reactor to which hydrogen and aromatic nitro compound are fed in a molar ratio of hydrogen to nitro group of 3:1 to 100:1.

**2.** Process according to claim 1, **characterised in that** the cell density of the monolithic support is 100 to 900 cpsi, preferably 200 to 600 cpsi.

**3.** Process according to one of claims 1 or 2, **characterised in that** the catalytically active coating has a film thickness

of no more than 250 μm, preferably of no more than 100 μm, particularly preferably of 10 to 100 μm.

4. Process according to one of claims 1-3, **characterised in that** the catalytically active coating contains platinum and/or palladium.

5. Process according to one of claims 1-4, **characterised in that** the catalytically active coating contains supported platinum and/or palladium.

6. Process according to one of claims 1-5, **characterised in that** the catalytic hydrogenation takes place under a pressure of 1 to 30 bar, preferably 1 to 20 bar, particularly preferably 1 to 15 bar.

7. Process according to one of claims 1-6, **characterised in that** the catalytic hydrogenation is carried out in two steps, wherein an adiabatically operated reactor with a catalyst consisting at least of a monolithic support and a catalytically active coating is used in the second step.

## Revendications

1. Procédé pour la préparation d'amines aromatiques par hydrogénation catalytique de composés nitro aromatiques, choisis parmi le nitrobenzène et les nitrotoluènes isomères, **caractérisé en ce que** l'hydrogénation catalytique est réalisée en au moins une étape et le catalyseur est constitué par au moins un support monolithique et par un revêtement catalytiquement actif, la température du mélange gazeux de départ, avant l'entrée dans le réacteur, étant de 200 à 400°C et l'hydrogénation ayant lieu à une température de 200 à 500°C dans un réacteur exploité de manière adiabatique alimenté en hydrogène et en composé nitro aromatique dans un rapport molaire d'hydrogène à groupe nitro de 3:1 à 100:1.

2. Procédé selon la revendication 1, **caractérisé en ce que** la densité cellulaire du support monolithique est de 100 à 900 cpsi, de préférence de 200 à 600 cpsi.

3. Procédé selon l'une quelconque des revendications 1 ou 2, **caractérisé en ce que** le revêtement catalytiquement actif présente une épaisseur de couche d'au maximum 250 μm, de préférence d'au maximum 100 μm, de manière particulièrement préférée de 10 à 100 μm.

4. Procédé selon l'une quelconque des revendications 1 à 3, **caractérisé en ce que** le revêtement catalytiquement actif contient du platine et/ou du palladium.

5. Procédé selon l'une quelconque des revendications 1-4, **caractérisé en ce que** le revêtement catalytiquement actif contient du platine et/ou du palladium supporté(s).

6. Procédé selon l'une quelconque des revendications 1 à 5, **caractérisé en ce que** l'hydrogénation catalytique a lieu à une pression de 1 à 30 bars, de préférence de 1 à 20 bars, de manière particulièrement préférée de 1 à 15 bars.

7. Procédé selon l'une quelconque des revendications 1-6, **caractérisé en ce que** l'hydrogénation catalytique est réalisée en deux étapes, un réacteur exploité de manière adiabatique, présentant un catalyseur, étant utilisé dans la deuxième étape, ledit catalyseur étant constitué par au moins un support monolithique et par un revêtement catalytiquement actif.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- US 3136818 A
- EP 0978505 A2
- EP 0696574 A
- DE 2849002 A
- WO 9825881 A
- GB 1452466 A
- DE 19931902 A
- EP 0696574 B
- WO 9846557 A


### In der Beschreibung aufgeführte Nicht-Patentliteratur


- **VON F. KAPTEIJN ; J. J. HEISZWOLF ; T. A. NIJHUIS ; J. A. MOULIJN.** Monoliths in multiphase catalytic processes - aspects and prospects. *Cattech,* 1999, vol. 3, 24
- **VON T. A. NIJHUIS ; A. E. W. BEERS ; T. VERGUNST ; I. HOEK ; F. KAPTEIJN ; J. A. MOULIJN.** Preparation of monolithic catalysts. *Catalysis Reviews,* 2001, vol. 43, 345-380